# EUROPÄISCHE PATENTSCHRIFT

(11) **EP 1 672 083 B1**
(45) Veröffentlichungstag und Bekanntmachung des Hinweises auf die Patenterteilung: **14.05.2008**
(21) Anmeldenummer: 05028091.6
(22) Anmeldetag: 10.02.1999
(51) Int. Cl.: C12Q 1/68, G02B 21/00

(54) **Diagnostischer Kit**
Diagnostic kit
Kit diagnostique

(30) Priorität: 16.02.1998 DE 19806303; 03.11.1998 DE 19850661
(43) Veröffentlichungstag der Anmeldung: 21.06.2006
(62) Teilanmeldung aus: 99101714.6
(73) Patentinhaber: MetaSystems Hard & Software GmbH, 68804 Altlussheim (DE)
(72) Erfinder: Chudoba, Ilse, Dr., 68804 Altlussheim (DE); Loerch, Thomas, Dr., 68799 Reilingen (DE); Plesch, Andreas, Dr., 68723 Schwetzingen (DE); Senger, Gabriele, Dr., 93055 Regensburg (DE)
(74) Vertreter: Müller-Boré & Partner Patentanwälte

(56) Entgegenhaltungen:
- WO-A1-97/40191
- MUELLER S ET AL: "Toward a multicolor chromosome bar code for the entire human karyotype by fluorescence in situ hybridization" HUMAN GENETICS, Bd. 100, Nr. 2, 1997, Seiten 271-278, XP002379315 ISSN: 0340-6717
- PINKEL D ET AL: "CYTOGENETIC ANALYSIS USING QUANTITATIVE, HIGH-SENSITIVITY, FLUORESCENCE HYBRIDIZATION" PROCEEDINGS OF THE NATIONAL ACADEMY OF SCIENCES OF USA, NATIONAL ACADEMY OF SCIENCE, WASHINGTON, DC, US, Bd. 83, Mai 1986 (1986-05), Seiten 2934-2938, XP002900461 ISSN: 0027-8424
- SPEICHER M R ET AL: "KARYOTYPING HUMAN CHROMOSOMES BY COMBINATORIAL MULTI-FLUOR FISH" NATURE GENETICS, NEW YORK, NY, US, Bd. 12, 12. April 1996 (1996-04-12), Seiten 368-375, XP000930084 ISSN: 1061-4036
- SCHROECK E ET AL: "MULTICOLOR SPECTRAL KARYOTYPING OF HUMAN CHROMOSOMES" SCIENCE, AMERICAN ASSOCIATION FOR THE ADVANCEMENT OF SCIENCE,, US, Bd. 273, 26. Juli 1996 (1996-07-26), Seiten 494-497, XP000952836 ISSN: 0036-8075

## Beschreibung

Die vorliegende Erfindung betrifft ein Verfahren zum Nachweis von Änderungen in Biopolymeren, insbesondere in chromosomaler DNA, unter Verwendung von zwei oder mehreren unterschiedlichen Sätzen von markierten Detektormolekülen sowie einen diagnostischen Kit zum Nachweis dieser Änderungen.

Die Darstellung menschlicher Chromosomen wird bisher mit Bänderungstechniken durchgeführt, die eine spezifische Erkennung der Chromosomen anhand von hellen und dunklen Banden erlauben (z.B. "G-banding, Q-banding, R-banding"). Diese Bänderungstechniken basieren auf Verfahren, die von Caspersson et al. (Exp. Cell Res. 60, 1970, 315-319), Sumner et al. (Nature 232, 1971, 31), Seabright et al. (Lancet 2, 1971, 971-972) und Dutrillaux et al. (C R Acad. Sci. Paris, 272, 1971, 3638-3640) entwickelt wurden. Mit diesen Verfahren kann jedoch die Identität einzelner chromosomaler Banden nicht in jedem Fall definiert werden, da alle Banden von allen Chromosomen lediglich entweder hell oder dunkel erscheinen. Dies erweist sich als ein wesentlicher Nachteil, da Chromosomen von Zelle zu Zelle und von Gewebe zu Gewebe morphologisch sehr unterschiedlich sein können und u.U. Translokationen (zum Beispiel bei Tumoren) aufweisen, deren Erkennung für die zu untersuchende Person von besonderer Bedeutung sein kann. Dies gilt beispielsweise für die Realisierung von Kinderwunsch bei balancierten Translokationen ("Chromosomenstückaustauschen") eines Elternteils, für die Erkennung der Ursache von Fehlbildungen bei Kindern mit und ohne geistige Retardierung und für die Diagnostik von Leukämien und anderer Tumoren, die häufig spezifische chromosomale Veränderungen von diagnostischer und therapeutischer Bedeutung aufweisen.

Als Vorschlag zur Lösung dieser Problematik wurde von Pinkel et al. (Proc. Natl. Acad. Sci. USA 83, 1986, 2934-2938) die Fluoreszenz-in-situ-Hybridisierung (FISH) erstmals für die Routine in praktikabler Form beschrieben. Mit diesem Verfahren können heute durch den Einsatz chromosomenspezifischer DNA-Banken alle menschlichen Chromosomen einer Metaphase in jeweils unterschiedlichen Farben dargestellt werden (chromosome painting, 24-colour-FISH, Schröck et al., Science 273, 1996, 496-497; Speicher et al. Nature Genet. 12, 1996, 368-375), und durch den Einsatz von Vektoren, beispielsweise Cosmiden, Pac's oder YAC's, die unterschiedliche Mengen an menschlicher DNA enthalten können, spezifische chromosomale Regionen erneut über Vielfarbentechniken mittels FISH in ihrer Integrität überprüft werden. Auch Teile von Genen und repetitive DNA-Elemente lassen sich über diesen Weg auf ihre chromosomale Lokalisation hin und auf ihr Vorhandensein bzw. Fehlen nachweisen. Eine mehrfarbige ("multicolor"-) Darstellung um chromosomalen Abschnitten auf Bandenniveau ist bisher jedoch nicht möglich.

WO 97/40191 beschreibt ein Spektralbildverfahren zum Nachweis und zur Analyse von verschiedenen Fluorochromen bei der Chromosomenfarbbänderung (vgl. beispielsweise "Abstract" und Anspruch 1) und Müller, S. et al., 1997 (Hum Genet (1997) 100:271-278) beschreibt ein Verfahren, worin zwei mit verschiedenen Fluorochromen markierte Sätze von subregionalen DNA-Sonden (DNA-Pools) auf menschlichen Metaphase-Chromosomen *in situ* hybridisiert werden (vgl. beispielsweise "Abstract").

Somit liegt der vorliegenden Erfindung die Aufgabe zugrunde, ein neues bzw. verbessertes Verfahren zum Nachweis von insbesondere Änderungen in chromosomaler DNA bereitzustellen, das eine mehrfarbige Darstellung auf Bandenniveau ermöglichen soll.

Diese Aufgabe wird durch die in den Ansprüchen gekennzeichneten Ausführungsformen der vorliegenden Erfindung gelöst.

Insbesondere wird eine Verwendung eines diagnostischen Kits, enthaltend mindestens zwei unterschiedliche Sätze von markierten Detektormolekülen, wobei jeweils mindestens zwei Sätze für einen bestimmten Bereich in den Zielmolekülen spezifisch sind und die Markierungen der jeweiligen Detektormoleküle dieser für einen bestimmten Bereich in den Zielmolekülen spezifischen Sätze unterschiedlich sind, in einem Verfahren zum Nachweis von Änderungen in Biopolymeren als Zielmoleküle, bereitgestellt, umfassend die Schritte:
(a) Durchführen von Bindungsreaktionen zwischen den Detektormolekülen der unterschiedlichen Sätze und den Zielmolekülen, wobei die jeweiligen markierten Detektormoleküle von mindestens zwei Sätzen derart an einen bestimmten Bereich der Zielmoleküle binden, daß sich die unterschiedlichen Markierungen der jeweiligen Detektormoleküle überlappen,
(b) Aufzeichnen der Intensitäten bzw. Intensitätsverhältnisse der unterschiedlichen Markierungen unter Verwendung einer Abtastvorrichtung, bei welcher die Intensitäten bzw. Intensitätsverhältnisse der Markierungen in den Bereichen von überlappenden und nicht-überlappenden Markierungen der jeweiligen Detektormoleküle in Längsrichtung der Zielmoleküle aufgezeichnet werden, und
(c) Auswerten der aufgezeichneten Intensitäten bzw. Intensitätsverhältnisse, wobei das Zielmolekül in mehrere kleine Abschnitte unterteilt wird und über ein geeignetes Rechenprogramm jedem dieser Abschnitte auf der Basis der relativen Intensitätverhältnisse eine Falschfarbe zugeordnet wird.

Der Begriff "Biopolymere als Zielmoleküle" bedeutet DNA, vorzugsweise chromosomale DNA, RNA oder Polypleptide. Die Zielmoleküle können vor Durchführung der erfindungsgemäßen Verwendung entsprechend angeordnet bzw. immobilisiert werden, beispielsweise durch gelelektrophoretische Auftrennung in einer geeigneten Matrix oder Fixierung bzw. Anordnung von beispielsweise Metaphase-Chromosomen oder Interphase-Kernen auf einen geeigneten Träger.

Der Begriff "markierte Detektormoleküle" bedeutet Nukleinsäuren oder Antikörper, die jeweils mindestens eine Markierung aufweisen. Die Antikörper können polyklonal oder monoklonal vorliegen. Die Begriffe "Nukleinsäure" bzw. "Nukleinsäuresequenz" bzw. "Nukleinsäuresonden" bedeuten native, halbsynthetische oder modifizierte Nukleinsäuremoleküle aus Desoxyribonukleotiden und/oder Ribonukleotiden und/oder modifizierten Nukleotiden wie Aminonukleotiden oder [α-S]-Triphosphatnukleotiden. In einer bevorzugten Ausführungsform der vorliegenden Erfindung stammen die Nukleinsäuren von chromosomaler DNA aus beispielsweise Säugern wie homo sapiens sapiens. Die chromosomale DNA als Detektormoleküle liegt in Vektoren, z.B. Cosmide oder YAC's vor, oder stammt aus chromosomalen bzw. Chromosomenregion-spezifischen DNA-Banken, die beispielsweise über Mikrodissektionsverfahren oder Laser aktivierte flußcytometrische Sortierung von spezifischen Chromosomen und, wenn erforderlich, nachfolgender Amplifikation durch beispielsweise DOP-PCR erhalten werden können.

Der Begriff "Markierung" bedeutet geeignete, direkt oder indirekt nachweisbare Atome oder Moleküle, die in die Detektormoleküle eingebaut oder damit verbunden sind. Geeignete Markierungen sind beispielsweise solche, die an Nukleotide gekoppelte Fluoreszenzfarbstoffe und/oder beispielsweise Biotin und/oder Digoxigenin und/oder mit radioaktiven Isotopen markierte Nukleotide umfassen. In einer bevorzugten Ausführungsform ist die Markierungsverbindung ein Fluoreszenzfarbstoff mit einer zur Selektion kleiner Substanzmengen ausreichendem Unterschied im Fluoreszenzverhalten der Emissionsspektren, wie z.B. Cumarine und Rodamine und/oder der Fluoreszenzlebensdauer wie, z.B. Fluoreszenzisothiocyanate und Europium-Chelat-markierte und/oder Porphirin-markierte Avidine.

Der Begriff "Bindungsreaktion" bedeutet eine Hybridisierung, vorzugsweise eine *in*-*situ*-Hybridisierung, oder eine Antigen/Antikörper-Reaktion, abhängig von der Wahl der Detektormoleküle und/oder der Zielmoleküle. Der Begriff "*in*-*situ*-Hybridisierung" bedeutet die Anlagerung einer synthetisch erzeugten und mit biologischen, physikalischen oder chemischen Markierungen zur Detektion versehenen DNA/RNA-Sonde als Detektormolekül an native in der Natur vorkommende DNA/RNA-Sequenzen, wobei die Anlagerung durch Denaturierung und Renaturierung der entsprechenden Nukleinsäuren erreicht wird. Selbstverständlich enthalten diese DNA/RNA-Sonden mindestens einen zur Hybridisierung an eine DNA/RNA-Sequenz des Zielmoleküls, wie ein Chromosom, befähigten Sequenzabschnitt. Dieser Sequenzabschnitt weist einen spezifischen, einzeln vorliegenden, vorzugsweise 100 bis 1.000 Basenpaare langen Sequenzbereich des Detektormoleküls auf, der sich an einen komplementären Bereich des Zielmoleküls bei einer geeigneten Temperatur, vorzugsweise bei 50°C oder weniger, und bei einer geeigneten Salzkonzentration, die vorzugsweise 50-300 mmol/l einwertige Ionen und 0-10 mmol/I zweiwertige Ionen enthält, unter Ausbildung von Wasserstoffbrücken anlagert. Die Bindungsreaktion der jeweiligen Sätze von markierten Detektormolekülen kann gleichzeitig oder aufeinanderfolgend durchgeführt werden.

Der Ausdruck "Satz von Detektormolekülen" bedeutet Detektormoleküle, die für einen bestimmten Bereich der Zielmoleküle spezifisch sind. Dieser Satz von Detektormolekülen kann beispielsweise in Vektoren vorliegende chromosomale DNA oder eine chromosomenspezifische DNA-Bank sein. Die Markierung der Detektormoleküle im Satz können gleich oder unterschiedlich sein, beispielsweise drei unterschiedliche Markierungen enthalten.

Der Ausdruck "mindestens zwei oder mehrere unterschiedliche Sätze von markierten Detektormolekülen" bedeutet das Vorliegen von mindestens einem Paar von unterschiedlichen Sätzen, wobei die Sätze dieses Paars in einem bestimmten Bereich bzw. Region der Zielmoleküle derart binden, daß sich mindestens die unterschiedlichen Markierungen der jeweiligen Detektormoleküle, vorzugsweise die Bindungsstellen der jeweiligen Detektormoleküle dieser unterschiedlichen Sätze überlappen. Diese erfindungsgemäße Eigenschaft von einem Paar unterschiedlicher Sätze bedeutet, daß die jeweiligen Detektormoleküle in den unterschiedlichen Sätzen eines Paars, welches aus diesem bestimmten Bereich der Zielmoleküle überlappend hergestellt bzw. gewonnen werden, als Standard bzw. zur vergleichenden Untersuchung mit entsprechend aufgearbeiteten Proben von Patienten verwendet werden können. In einer erfindungsgemäßen Ausführungsform sind die Detektormoleküle eines Satzes vorzugsweise derart gestaltet, daß nach der Hybridisierung die Detektormoleküle in kontinuierlich veränderter Konzentration, vorzugsweise in Art einer Gaußschen Verteilung, in Längsrichtung an die Zielmoleküle, beispielsweise Chromosomen, gebunden sind.

Die qualitative und quantitative Auswertung der erhaltenen Bindungen über die unterschiedlichen Markierungen der Detektormoleküle kann unter Verwendung einer Abtasteinrichtung bzw. einer Vorrichtung zum gerichteten Abtasten, beispielsweise entlang bzw. in Längsrichtung des zu untersuchenden Chromosoms, verwendet werden. Eine derartige Abtasteinrichtung ist beispielsweise ein Fluoreszenzmikroskop. Über die physikalischen und/oder chemischen und/oder biologischen Markierungen der Detektormoleküle, die sich an die gewünschten Zielmoleküle angelagert haben, können durch die Abtasteinrichtung bilderzeugende Signale über eine Bildverarbeitungseinheit, beispielsweise eine CCD-Kamera, aufgenommen werden, welche computergestützt die einzelnen Signale der unterschiedlichen Markierungen in geeigneter Weise verarbeitet. Mit dieser an die Abtastvorrichtung gekoppelten Bildverarbeitungseinheit können die Intensitäten bzw. die Intensitätsverhältnisse der unterschiedlichen Markierungen in den Bereichen von überlappenden und nicht-überlappenden Markierungen der jeweiligen Detektormoleküle vorzugsweise in Längsrichtung der Zielmoleküle, insbesondere von fixierten Metaphase-Chromosomen, aufgezeichnet und qualitativ sowie quantitativ ausgewertet werden.

Die erfindungsgemäße Verwendung kann insbesondere zum Nachweis bzw. Ausschluß von chromosomalen Abänderungen in der Humangenetik, wie balancierter Chromosomenrearrangements, die bekanntermaßen von großer Bedeutung für die Verwirklichung von Kinderwunsch bei Trägern einer solchen Veränderung sind, balancierter und unbalancierter Chromosomenveränderungen als Ursache für Fehlbildungen und/oder mentaler Retardierung, und in der Tumordiagnostik sowohl solider Tumore als auch hämatologischer Neoplasien (AML, ALL, MDS u.a.) einerseits zur Erfassung bekannter, prognoserelevanter Veränderungen andererseits zur Bestimmung weiterer, bisher unbekannter Veränderungen eingesetzt werden.

Ein weiterer Gegenstand der vorliegenden Erfindung ist eine automatische Korrektur durch Hinzufügen einer lokalisierten DNA-Sonde.

Bei der Aufnahme von mit verschiedenen Fluorochromen markierten chromosomenregion-spezifischen Proben, wie sie für die Methode des Multi Color Banding eingesetzt werden, wird eine Monochrom-CCD Kamera in Kombination mit spezifischen Fluoreszenzfiltern verwendet. Die Signale der einzelnen Fluorochrome werden nacheinander als Einzelbilder aufgenommen und anschließend zu einem Farbbild zusammengesetzt. Eine Verschiebung der Lage der Einzelbilder gegeneinander aufgrund optischer Einflüsse der Filter (unterschiedliche Keilfehler, Parallelversatz durch geringfügige Verkippung im Strahlengang) ist dabei nicht auszuschließen. Eine interaktive oder automatische Korrektur, etwa durch eine Korrelation der Einzelbilder, ist nicht mit der erforderlichen Präzision möglich, da die allenfalls teilweise überlappenden Sonden keine gemeinsamen Strukturen aufweisen, die für eine nachträgliche Überlagerung genutzt werden können. Jede geringfügige Verschiebung führt bei der Auswertung der Intensitätsverhältnisse zu Artefakten im Bänderungsmuster.

Die automatische Korrekur wird durch Beifügen einer lokalisierten DNA-Sonde ermöglicht, die mit allen im erfindungsgemäßen Verfahren verwendeten Fluorochromen gleichzeitig markiert ist. Damit steht eine in allen Einzelbildern identische Struktur zur automatischen Lagekorrektur zur Verfügung. Die Lagekorrektur kann z. B. über eine Ermittlung des Intensitätsschwerpunkts der Sonde in jedem Einzelbild und anschließend relativer Verschiebung der Einzelbilder so erfolgen, daß die Schwerpunkte der Einzelbilder auf die gleiche Position zu liegen kommen.

Durch den Einsatz zweier verschiedener Sonden können auch Verdrehungen der Bilder gegeneinander ermittelt und korrigiert werden.

Der Einsatz von noch mehr Sonden ermöglicht grundsätzlich die Korrektur von komplexeren Lagetransformationen als Translation und Rotation, wie z. B. Maßstabsveränderungen.

Ferner kann es in einer weiteren bevorzugten Ausführungsform der vorliegenden Erfindung von Vorteil sein, Kalibriersonden (DNA-Sonden oder fluoreszierende Partikel) bekannter Intensität beizufügen, die zur Normierung der Intensitäten der auszuwertenden Fluoreszenzsignale dienen können.

Ferner kann es in einer weiteren bevorzugten Ausführungsform der vorliegenden Erfindung von Vorteil sein, DNA-Sonden beizufügen, deren genaue Lokalisation innerhalb des Genoms bekannt sind, und die dazu verwendet werden können, den Bezug zwischen Farbbanden und den ISCN-Banden herzustellen.

Die Figuren zeigen:

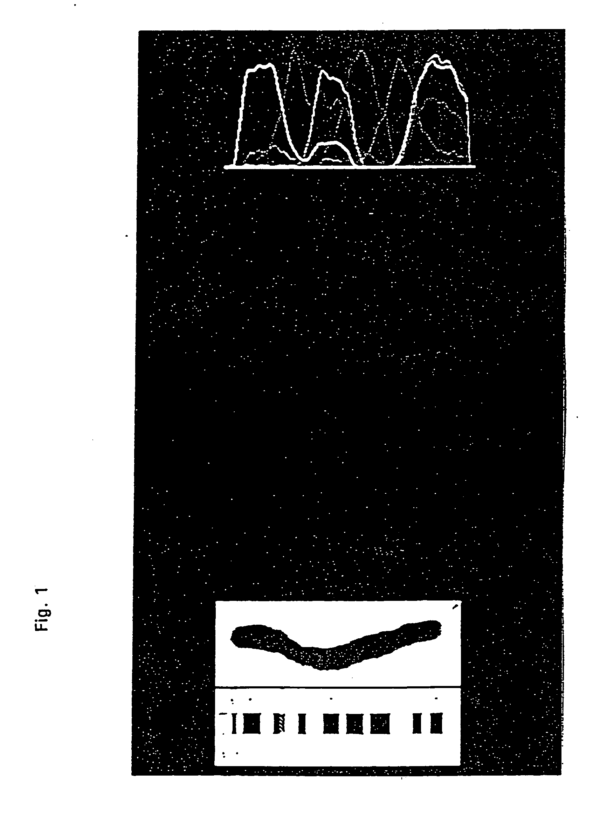

- Fig. 1: ist eine photographische Darstellung zur qualitativen und quantitativen Auswertung der Lokalisation von Region-spezifischen Färbungen in Chromosom 5. Im oberen Teil dieser Figur ist die Verteilung der markierten Detektormoleküle in Längsrichtung des Chromosoms sowie Intensitäten der unterschiedlichen Markierungen der Detektormoleküle graphisch dargestellt.

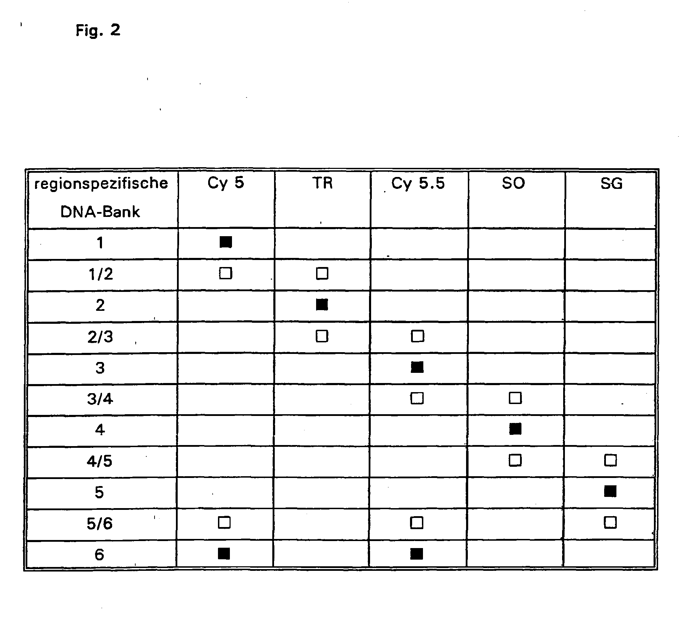

- Fig. 2: ist eine tabellarische Darstellung des Markierungsmusters des in Fig. 1 dargestellten Region-spezifischen Chromosomenabschnitts von Chromosom 5. Cy5, TR(Texas Red), Cy5.5, SO (Spectrum Orange), SG (Spectrum Green) sind die verschiedenen FluoreszenzFarbstoffe, die zum Markieren der einzelnen regionspezifischen DNA-Banken verwendet wurden. Die Zuordnung ist durch eine ausgefülltes Quadrat (■) gekennzeichnet. Die durch die Überlappung der DNA-Banken sich ergebende Markierungen in den entsprechenden Bereichen ist durch ein leeres Quadrat (□) kenntlich gemacht.
- Fig. 3: zeigt jeweils die homologen normalen Chromosomen 5 aus zwei verschiedenen Metaphaseplatten mit mehrfarbiger Bänderung. Die Darstellung macht deutlich, daß das Bänderungsmuster auf den homologen Chromosomen identisch ist und auch von Metaphaseplatte zu Metaphaseplatte reproduzierbar ist.

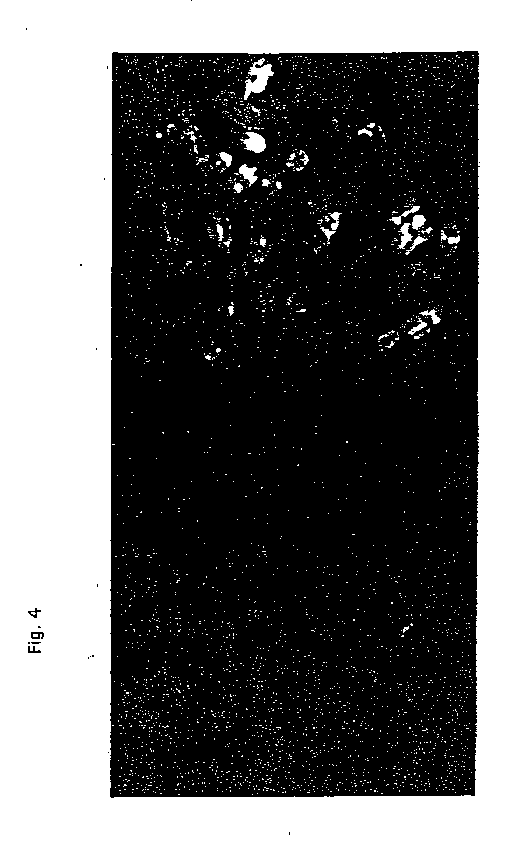

- Fig. 4: zeigt eine photographische Darstellung einer Vielfarben-FISH einer Metaphaseplatte mit komplexen chromosomalen Abänderungen.
- Fig. 5: zeigt Chromosomen 5 in einem Fall mit akuter myeloischer Leukämie. Auf der rechten Seite ist jeweils das normale Chromosom 5 dargestellt, das Chromosom auf der linken Seite zeigt eine interstitielle Deletion im langen Arm.

Das folgende Beispiel erläutert die Erfindung.

### Beispiel

Für das Vielfarben-Bandenmuster von Chromosom 5 wurden insgesamt 7 überlappende Chromosomenregion-spezifische Mikrosezierungsbanken hergestellt (Meltzer et al., Nature Genet. 1, 1992, 24-28). Der p-Arm von Chromosom 5 wurde hierfür in zwei, der q-Arm in vier Regionen unterteilt. Pro Chromosomenregion wurden jeweils 8-10 Fragmente mit einer fein ausgezogenen Glasnadel vom Objektträger unter mikroskopischer Sicht isoliert (Senger et al., Hum. Genet. 84, 1990, 507-511). Die so gewonnene DNA wurde über eine DOP-PCR (degenerate oligonucleotide polymerase chain reaction, Telenius et al., Genomics 13, 1992, 718-725; Zhang et al., Blood 81, 1993, 3365-3371) amplifiziert. In einer Folgereaktion wurden diese Chromosomenregion-spezifischen DNA-Banken teilweise direkt mit Fluorochromen, die an Nukleotide gekoppelt vorliegen, markiert (z.B. Spectrum Orange-dUTP, Spectrum Green-dUTP, beide Vysis, und Texas Red-dUTP, Molecular Probe). Zum anderen Teil wurden DNA-Banken mit Nukleotiden markiert, die mit Haptenen (z.B. Biotin-dUTP und DigoxigenindUTP, Boehringer, Mannheim) gekoppelt sind. Nach erfolgter Hybridisierung können Haptene mit geeigneten Detektionsreagenzien (z.B. Avidin-Cy5, Amersham und Anti-Digoxigenin IgG, Boehringer, Mannheim, der an Cy5.5 gekoppelt ist, Mab-Iabeling kit, Amersham) detektiert werden.

Die Hybridisierung, Waschschritte und Detektion erfolgen nach Standardprotokollen (Senger et al., Cytogenet. Cell. Genet. 64, 1993, 49-53).

Die Analyse wird z.B. mit einem Fluoreszenzmikroskop durchgeführt, das mit geeigneten Filtersätzen ausgestattet ist. Von jedem Farbkanal werden separat Bilder aufgenommen, die anschließend mit einem Computer weiterbearbeitet werden können.

Ein charakteristisches Merkmal der Teil-"painting"-Sonden, die durch Mikrosezierung gewonnen werden, ist ein sich kontinuierlich abschwächendes Fluoreszenzsignal in den Randbereichen. Durch gleichzeitiges Überlappen der Sonden und damit der Fluoreszenzsignale zweier benachbarter Teil-"painting"-Sonden kommt es zu einem sich kontinuierlich verändernden Verhältnis der Fluoreszenzintensitäten entlang von Chromosom 5. Wird ein so gefärbtes Chromosom in mehrere (20-25) kleine Abschnitte unterteilt, so kann über ein geeignetes Rechenprogramm jedem dieser Sektoren auf der Basis der relativen Fluoreszenzintensitäten aller verwendeten Fluorchrome eine Falschfarbe zugeordnet werden. Durch diese Zuordnung kommt es zu einem farblichen Bandenmuster entlang eines Chromosoms, in diesem Fall von Chromosom 5. Bei allen weiteren Hybridisierungen mit dem selben Probenset kann die gleiche Kombination von Fluoreszenzverhältnissen und Falschfarben verwendet werden.

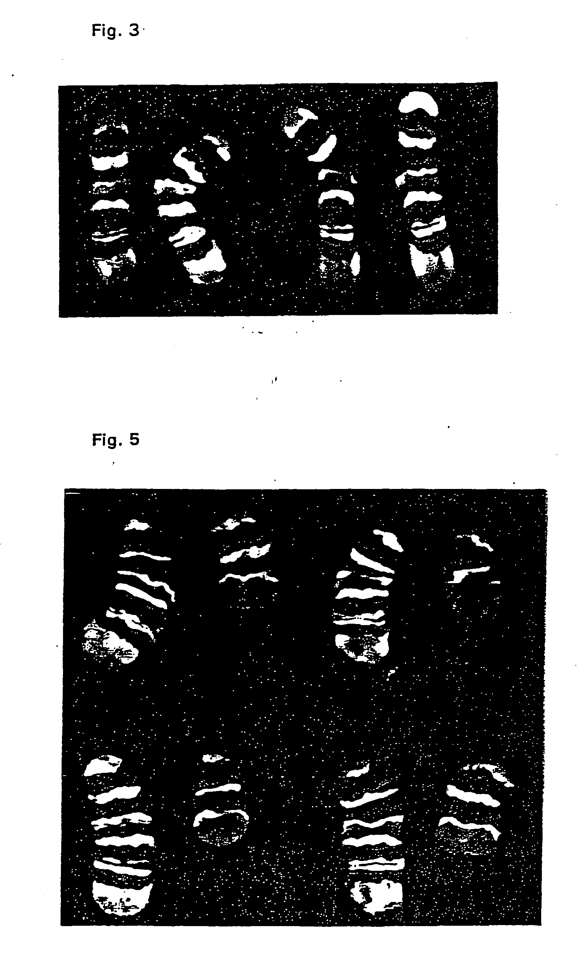

Da die Hybridisierung sich ausreichend konstant verhält, ist auch das Bandenmuster entsprechend reproduzierbar (Figur 3). Ein Verlust des Auflösungsvermögens bei kürzeren Chromosomen, wie es von bislang üblichen Bänderungsverfahren (z.B. GTG-Bänderung) bekannt ist, wird hierbei nicht beobachtet. Für das Chromosom 5 wird ein reproduzierbares Muster von mindestens 25 Banden erreicht. Dies entspricht einem Bandenniveau von ca. 550 Banden pro haploiden Chromosomensatz.

Mit Hilfe dieses Verfahrens ist es möglich Veränderungen an Chromosomen nachzuweisen unabhängig von ihrem Kondensationszustand. Dies ist vor allem auch in der Tumorzytogenetik von Bedeutung. Tumorchromosomen zeigen oft eine geringe Auflösung des Bandenmusters, wodurch ein Erkennen von chromosomalen Veränderungen wesentlich erschwert ist. Es ist daher anzunehmen, daß bislang unbekannte zytogenetische Veränderungen in Tumoren vorliegen, die möglicherweise einen wichtigen Prognosefaktor darstellen und daher z.B. für eine risikoangepaßte Therapie von Bedeutung sein könnten. Erfindungsgemäß können auch an Tumorchromosomen nach Hybridisierung mit dem oben näher beschriebenen Probenset für das Chromosom 5 mindestens 25 Banden erreicht werden (Figur 5).

## Patentansprüche

1. Verwendung eines diagnostischen Kits, enthaltend mindestens zwei unterschiedliche Sätze von markierten Detektormolekülen, wobei jeweils mindestens zwei Sätze für einen bestimmten Bereich in den Zielmolekülen spezifisch sind und die Markierungen der jeweiligen Detektormoleküle dieser für einen bestimmten Bereich in den Zielmolekülen spezifischen Sätze unterschiedlich sind, in einem Verfahren zum Nachweis von Änderungen in Biopolymeren als Zielmoleküle, umfassend die Schritte:
(a) Durchführen von Bindungsreaktionen zwischen den Detektormolekülen der unterschiedlichen Sätze und den Zielmolekülen, wobei die jeweiligen markierten Detektormoleküle von mindestens zwei Sätzen derart an einen bestimmten Bereich der Zielmoleküle binden, daß sich die unterschiedlichen Markierungen der jeweiligen Detektormoleküle überlappen,
(b) Aufzeichnen der Intensitäten bzw. Intensitätsverhältnisse der unterschiedlichen Markierungen unter Verwendung einer Abtastvorrichtung, bei welcher die Intensitäten bzw. Intensitätsverhältnisse der Markierungen in den Bereichen von überlappenden und nicht-überlappenden Markierungen der jeweiligen Detektormoleküle in Längsrichtung der Zielmoleküle aufgezeichnet werden, und
(c) Auswerten der aufgezeichneten Intensitäten bzw. Intensitätsverhältnisse, wobei das Zielmolekül in mehrere kleine Abschnitte unterteilt wird und über ein geeignetes Rechenprogramm jedem dieser Abschnitte auf der Basis der relativen Intensitätverhältnisse eine Falschfarbe zugeordnet wird.

2. Verwendung nach Anspruch 1, wobei die markierten Detektormoleküle aus Nukleinsäuren oder Antikörpern ausgewählt sind.

3. Verwendung nach Anspruch 2, wobei die jeweiligen Sätze von Nukleinsäuren von unterschiedlichen Chromosomenregion-spezifischen DNA-Banken stammen.

4. Verwendung nach einem der Ansprüche 1 bis 3, wobei jeder Satz von Detektormolekülen ein oder mehrere unterschiedliche Markierungen enthält.

5. Verwendung nach Anspruch 4, wobei die Markierung einen Fluoreszenzfarbstoff umfasst.

6. Verwendung nach einem der Ansprüche 1 bis 5, wobei mindestens eine mit mindestens zwei der verwendeten N Fluorochome markierte DNA-Sonde zur Ermittlung einer optisch verursachten relativen Verschiebung der Bildinformation der Fluorochrome und zu ihrer Lagekorrektur beigefügt ist.

7. Verwendung nach Anspruch 6, wobei mindestens eine mit allen N verwendeten Fluorochromen markierte DNA-Sonde zur gleichzeitigen Lagekorrektur aller Fluorochrome beigefügt ist.

8. Verwendung nach Anspruch 6, wobei N-1 DNA-Sonden mit je zwei geeigneten Fluorochromen markiert sind und die Lagekorrektur paarweise durchgeführt wird.

9. Verwendung nach einem der Ansprüche 1 bis 8, wobei Kalibriersonden bekannter bzw. reproduzierbarer konstanter Intensität zur Normierung der Signalintensitäten beigefügt sind.

10. Verwendung nach Anspruch 9, wobei die Kalibriersonden fluoreszenzmarkierte DNA-Sonden sind.

11. Verwendung nach Anspruch 9, wobei die Kalibriersonden fluoreszenzmarkierte Partikel sind.

12. Verwendung nach einem der Ansprüche 1 bis 11, wobei zusätzliche DNA-Sonden für eine direkte Zuordnung von Farbbanden zur ISCN Nomenklatur beigefügt sind.

13. Verwendung nach einem der Ansprüche 6 bis 12, wobei diese Sonden gleichzeitig zur Lagekorrektur und/oder Intensitätnormierung verwendet werden.

14. Verwendung nach einem der Ansprüche 1 bis 13 zum Nachweis von chromosomalen Abänderungen in der Humangenetik und Tumordiagnostik.

## Claims

1. A use of a diagnostic kit, containing at least two different sets of labeled detector molecules, wherein at least two sets are specific for a certain region in the target molecules and the labels of the particular detector molecules of said sets, being specific for a certain region in the target molecule, are different, in a method for detecting changes in biopolymers as target molecules comprising the steps of:
(a) carrying out binding reactions between the detector molecules of the different sets and the target molecules, wherein the particular labeled detector molecules of at least two sets bind in such a manner to a certain region of the target molecules that the different labels of the particular detector molecules overlap,
(b) recording the intensities and intensity ratios, respectively, of the different labels using a sampling device, wherein the intensities and intensity ratios, respectively, of the labels in the regions of overlapping and nonoverlapping labels of the particular detector molecules are recorded in longitudinal direction of the target molecules, and
(c) evaluating the recorded intensities and intensity ratios, respectively, wherein the target molecule is divided into a plurality of small sections and, using a suitable computer program, each of said sections is assigned with a false color based on the relative intensity ratios.

2. The use according to claim 1, wherein the labeled detector molecules are selected from nucleic acids or antibodies.

3. The use according to claim 2, wherein the particular sets of nucleic acids originate from different chromosome region-specific DNA libraries.

4. The use according to any of claims 1 to 3, wherein each set of detector molecules contains one or more different labels.

5. The use according to claim 4, wherein said label comprises a fluorescence dye.

6. The use according to any of claims 1 to 5, wherein at least one DNA probe being labeled with at least two of the used N fluorochromes is added to determine an optically caused relative shift of the image information of the fluorochromes and for its positional correction.

7. The use according to claim 6, wherein at least one DNA probe being labeled with all of the used N fluorochromes is added for the simultaneous positional correction of all fluorochromes.

8. The use according to claim 6, wherein N-1 DNA probes are each labeled with two suitable fluorochromes and the positional correction is carried out pair-wise.

9. The use according to any of claims 1 to 8, wherein calibrating probes of known and reproducible constant intensity, respectively, are added for normalization of the signal intensities.

10. The use according to claim 9, wherein the calibrating probes are fluorescence-labeled DNA probes.

11. The use according to claim 9, wherein the calibrating probes are fluorescence-labeled particles.

12. The use according to any of claims 1 to 11, wherein additional DNA probes for a direct assignment of color bands to the ISCN nomenclature are added.

13. The use of any of claims 6 to 12, wherein the probes are used simultaneously for positional correction and/or intensity normalization.

14. The use according to any of claims 1 to 13, for the detection of chromosomal aberrations in human genetics and tumor diagnostics.

## Revendications

1. Utilisation d'un kit de diagnostic, contenant au moins deux lignes différentes de molécules détectrices marquées, dans laquelle respectivement au moins deux lignes sont spécifiques à une zone déterminée dans les molécules cibles et les marquages des molécules détectrices respectives de ces lignes spécifiques à une zone déterminée dans les molécules cibles sont différents, dans un procédé de détection des modifications de biopolymères comme molécules cibles, incluant les étapes de :
(a) réalisation de réactions de liaison entre les molécules détectrices des différentes lignes et les molécules cibles, dans laquelle les molécules détectrices marquées respectives d'au moins deux lignes se lient à une zone déterminée des molécules cibles de telle sorte que les différents marquages des molécules détectrices respectives se chevauchent,
(b) enregistrement des intensités ou des rapports d'intensité des différents marquages par utilisation d'un dispositif de balayage par lequel sont enregistrés les intensités ou respectivement les rapports d'intensité des marquages dans les zones de marquage chevauchant et non-chevauchant des molécules détectrices respectives suivant la longueur des molécules cibles, et
(c) interprétation des intensités ou respectivement des rapports d'intensités enregistrés, dans laquelle la molécule cible est subdivisée en plusieurs petites sections et se voit octroyer pour chacune de ces sections une fausse couleur sur la base des rapports d'intensité relatifs par un programme de calcul adéquat.

2. Utilisation selon la revendication 1, dans laquelle les molécules détectrices marquées sont sélectionnées parmi les acides nucléiques ou les anticorps.

3. Utilisation selon la revendication 2, dans laquelle les lignes respectives d'acides nucléiques proviennent de banques d'ADN spécifiques à différentes régions chromosomiques.

4. Utilisation selon une des revendications 1 à 3, dans laquelle chaque ligne de molécules détectrices contient un ou plusieurs marquages différents.

5. Utilisation selon la revendication 4, dans laquelle le marquage comprend un colorant fluorescent.

6. Utilisation selon une des revendications 1 à 5, dans laquelle au moins une sonde d'ADN marquée par au moins deux des N fluorochromes utilisés est adjointe pour déterminer un décalage relatif d'origine optique de l'information graphique des fluorochromes et pour la correction de leur position.

7. Utilisation selon la revendication 6, dans laquelle au moins une sonde d'ADN marquée par tous les N fluorochromes utilisés est adjointe pour corriger en même temps la position de tous les fluorochromes.

8. Utilisation selon la revendication 6, dans laquelle N-1 sondes d'ADN sont marquées chacune par deux fluorochromes adéquats et la correction de position est réalisée par paires.

9. Utilisation selon une des revendications 1 à 8, dans laquelle des sondes de calibrage à intensité constante connue ou reproductible sont adjointes pour normaliser l'intensité des signaux.

10. Utilisation selon la revendication 9, dans laquelle les sondes de calibrage sont des sondes d'ADN marquées par fluorescence.

11. Utilisation selon la revendication 9, dans laquelle les sondes de calibrage sont des particules marquées par fluorescence.

12. Utilisation selon une des revendications 1 à 11, dans laquelle des sondes d'ADN supplémentaires sont adjointes pour permettre une association directe de bandes colorées à la nomenclature ISCN.

13. Utilisation selon une des revendications 6 à 12, dans laquelle ces sondes sont en même temps utilisées pour corriger la position et/ou pour normaliser l'intensité.

14. Utilisation selon une des revendications 1 à 13 pour la détection de modifications chromosomiques en génétique humaine et dans le diagnostic des tumeurs.
